# EUROPEAN PATENT APPLICATION

(11) **EP 4 775 895 A1**
(43) Date of publication of application: **15.07.2026**
(21) Application number: 26150364.3
(22) Date of filing: 06.01.2026
(51) Int. Cl.: F24F 11/00, F24F 12/00

(54) **INTELLIGENT NETWORKED ENERGY RECOVERY VENTILATOR**

(30) Priority: 13.01.2025 TW 114101347
(71) Applicant: Microjet Technology Co., Ltd., Hsinchu (TW)
(72) Inventor: Mou, Hao-Jan, Hsinchu (TW); Wu, Chin-Chuan, Hsinchu (TW); Huang, Chi-Feng, Hsinchu (TW)
(74) Representative: Uexküll & Stolberg

(57) **Abstract**

An intelligent networked energy recovery ventilator includes a main body (1), a filtering component (2), an air guiding fan (3), a heat exchange core (4), a host driving controller (5) and a gas detection module (6). The gas detection module (6) is disposed in the main body (1) and electrically connected to the host driving controller (5). The gas detection module (6) detects temperature, humidity and air pollution in the air to generate detection data, the detection data is transmitted to a networked cloud computing service device (7) through IoT communication, and the networked cloud computing service device (7) collects, analyzes and monitors the detection data in real time and intelligently selects a control instruction for transmitting to the gas detection module (6) to control the host driving controller (5) to enable the air guiding fan (3) and dynamically adjust operation frequency and output air volume of the air guiding fan (3), thereby achieving gas exchange and heat exchange between indoor field and outdoor field.

## Description

### FIELD OF THE INVENTION

The present disclosure relates to an air conditioning and ventilation technology, and more particular to an intelligent networked energy recovery ventilator capable of maintaining air circulation while recovering and conditioning the heat and the humidity for improving the performance and the indoor air quality.

### BACKGROUND OF THE INVENTION

Typically, conventional energy recovery ventilators cannot automatically adjust ventilation volume and heat recovery efficiency according to indoor and outdoor environments, and lack the function of remote control. With the growth of intelligent home energy-saving demands, the demand for intelligent and high-efficient energy recovery ventilator is also gradually increased. The present disclosure provides an intelligent networked energy recovery ventilator capable of detecting and analyzing environmental data in real time and realizing remote monitoring and operation through networking technology. Therefore, it is necessary to develop an intelligent networked energy recovery ventilator which can monitor environmental data in real time, intelligently condition indoor air quality, temperature and humidity and is equipped with remote controlling function.

### SUMMARY OF THE INVENTION

One object of the present disclosure is to provide an intelligent networked energy recovery ventilator, which includes a built-in gas detection module for detecting indoor and outdoor air qualities in real time. Moreover, the gas detection module is equipped with cloud connection capability, so that the user can remotely monitor environmental condition and control the equipment. Furthermore, through IoT (Internet of Things) communication, the ventilation volume and heat recovery efficiency can be dynamically adjusted based on detection data, such as temperature, humidity and air quality, thereby maintaining an optimal indoor air quality.

In accordance with an aspect of the present disclosure, an intelligent networked energy recovery ventilator is provided. The intelligent networked energy recovery ventilator includes a main body comprising an intake channel and an exhaust channel; at least one filtering component disposed at an entrance of the intake channel and/or an entrance of the exhaust channel; at least one air guiding fan disposed at a rear side of a filtering component of the at least one filtering component at the entrance of the intake channel and/or at a rear side of a filtering component opposite of the at least one filtering component at the entrance of the exhaust channel, wherein the at least one air guiding fan is configured to guide a gas to pass through the at least one filtering component for purification and to achieve a gas exchange between an indoor field and an outdoor field; a heat exchange core disposed in the main body and respectively communicated with the intake channel and the exhaust channel, wherein the intake channel and the exhaust channel are not communicated with each other, the intake channel is separated by the heat exchange core to communicate with the indoor field through one end thereof and with the outdoor field through the other end thereof, and the exhaust channel is separated by the heat exchange core to communicate with the indoor field through one end thereof and with the outdoor field through the other end thereof, so that a gas is guided to pass through the heat exchange core for achieving a heat exchange, and wherein the intake channel guides a gas from the outdoor field to the indoor field, and the exhaust channel guides a gas from the indoor field to the outdoor field, so as to achieve the gas exchange between the indoor field and the outdoor field; at least one host driving controller controlling an enablement of the at least one air guiding fan, and dynamically adjusting an operation frequency and an output air volume of the at least one air guiding fan; and at least one gas detection module electrically connected to the at least one host driving controller, wherein the at least one gas detection module detects a temperature, a humidity and an air pollution in an air to generate a detection data, the detection data is transmitted to a networked cloud computing service device through IoT communication, and the networked cloud computing service device collects and analyzes the detection data in real time, intelligently selects a control instruction according to the detection data, and transmits the control instruction to the at least one gas detection module, for controlling the at least one host driving controller to enable the at least one air guiding fan in the intake channel and/or the exhaust channel and dynamically adjust the operation frequency and the output air volume of the at least one air guiding fan, thereby achieving the gas exchange and the heat exchange between the indoor field and the outdoor field.

### BRIEF DESCRIPTION OF THE DRAWINGS

The above contents of the present disclosure will become more readily apparent to those ordinarily skilled in the art after reviewing the following detailed description and accompanying drawings, in which:
FIG. 1 is a schematic cross-sectional view illustrating an intelligent networked energy recovery ventilator according to an embodiment of the present disclosure;
FIG. 2 is a schematic diagram illustrating the controlling architecture of a gas detection module of the intelligent networked energy recovery ventilator according to the embodiment of the present disclosure;
FIG. 3 is a schematic cross-sectional view illustrating the assembly of a filtering component according to the embodiment of the present disclosure;
FIG. 4A is a schematic perspective view illustrating a gas detection main part of the gas detection module according to the embodiment of the present disclosure;
FIG. 4B is another schematic perspective view illustrating the gas detection main part of the gas detection module according to the embodiment of the present disclosure from another view angle;
FIG. 5 is an exploded view illustrating the gas detection main part of the gas detection module according to the embodiment of the present disclosure;
FIG. 6A is a schematic perspective view illustrating a base of the gas detection main part of the gas detection module according to the embodiment of the present disclosure;
FIG. 6B is another schematic perspective view illustrating the base of the gas detection main part of the gas detection module according to the embodiment of the present disclosure from another view angle;
FIG. 6C is a schematic view illustrating the base combined with a laser component and a piezoelectric actuator separated from the base of the gas detection main part of the gas detection module according to the embodiment of the present disclosure;
FIG. 7 a schematic perspective view illustrating the combination of the piezoelectric actuator of the gas detection main part of the gas detection module and the base according to the embodiment of the present disclosure;
FIG. 8A is a schematic exploded view illustrating the piezoelectric actuator of the gas detection main part of the gas detection module according to the embodiment of the present disclosure;
FIG. 8B is another schematic exploded view illustrating the piezoelectric actuator of the gas detection main part of the gas detection module according to the embodiment of the present disclosure from another view angle;
FIG. 9A is a schematic cross-sectional view illustrating the piezoelectric actuator of the gas detection main part of the gas detection module according to the embodiment of the present disclosure;
FIG. 9B is a schematic cross-sectional view illustrating an action of the piezoelectric actuator of the gas detection main part of the gas detection module according to the embodiment of the present disclosure;
FIG. 9C is a schematic cross-sectional view illustrating another action of the piezoelectric actuator of the gas detection main part of the gas detection module according to the embodiment of the present disclosure;
FIG. 10A is a schematic cross-sectional view illustrating the gas introduction in the gas detection main part of the gas detection module according to the embodiment of the present disclosure;
FIG. 10B is a schematic cross-sectional view illustrating the gas detection in the gas detection main part of the gas detection module according to the embodiment of the present disclosure;
FIG. 10C is a schematic cross-sectional view illustrating the gas exhaustion in the gas detection main part of the gas detection module according to the embodiment of the present disclosure; and
FIG. 11 is a schematic diagram illustrating the architecture of a networked cloud computing service device according to the embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this disclosure are presented herein for purpose of illustration and description only. It is not intended to be exhaustive or to be limited to the precise form disclosed.

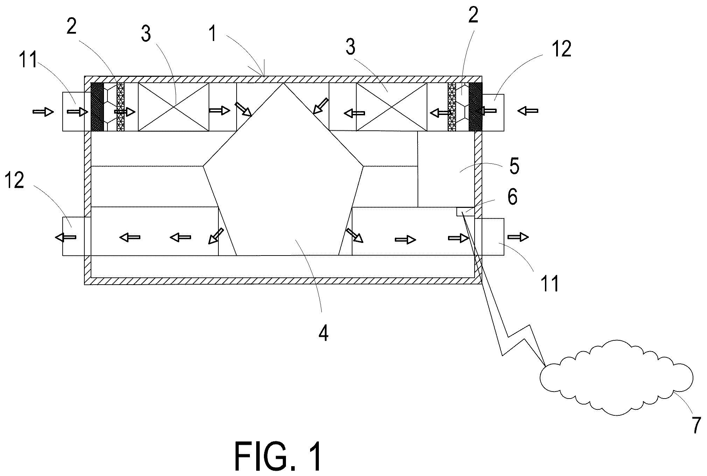

Please refer to FIG. 1. The present disclosure provides an intelligent networked energy recovery ventilator includes a main body 1, at least one filtering component 2, at least one air guiding fan 3, a heat exchanging core 4, at least one host driving controller 5 and at least one gas detection module 6. It is worth noting that in the embodiment of the drawings, there are two filtering components 2, two air guiding fans 3, one host driving controller 5 and one gas detection module 6, but the present disclosure is not limited thereto. The numbers thereof can be varied in accordance with practical requirements.

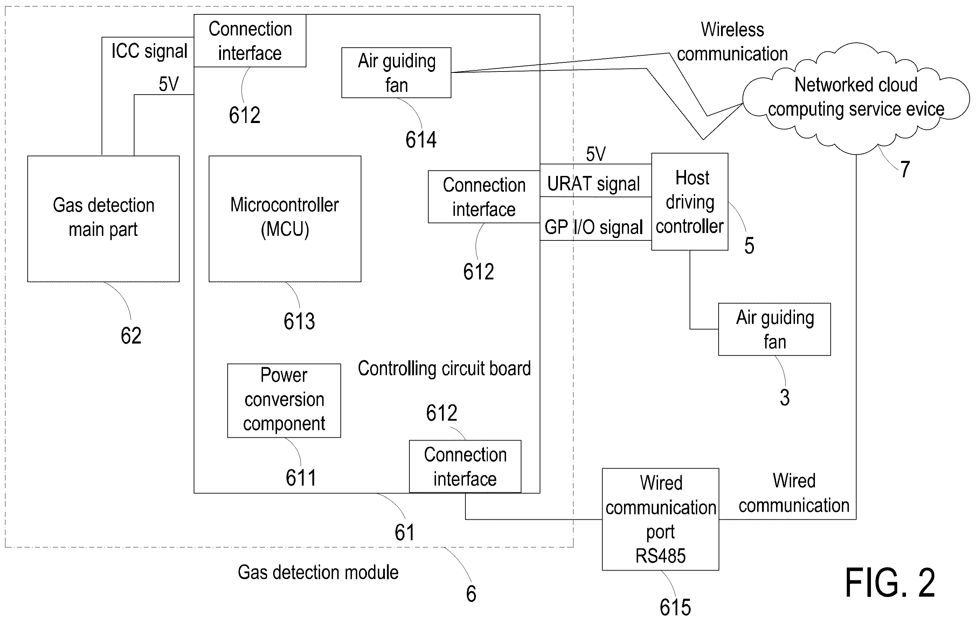

In the embodiment, the main body 1 includes an intake channel 11 and an exhaust channel 12. The filtering components 2 are respectively disposed at the entrances of the intake channel 11 and the exhaust channel 12. The air guiding fans 3 are respectively disposed at the rear side of the filtering components 2 at the entrances of the intake channel 11 and the exhaust channel 12. The air guiding fans 3 guide the air to pass through the filtering components 2 for purification, and also to achieve a gas exchange between the indoor field and the outdoor field. The heat exchanging core 4 is disposed inside the main body 1 and is fluidly communicated with the intake channel 11 and the exhaust channel 12 while the intake channel 11 and the exhaust channel 12 are not fluidly communicated with each other, wherein the intake channel 11 is divided by the heat exchanging core 4 to fluidly communicate with the outdoor field through one end and with the indoor field through the other end, and the exhaust channel 12 is divided by the heat exchanging core 4 to fluidly communicate with the outdoor field through one end and with the indoor field through the other end, so as to achieve a heat exchange. The intake channel 11 guilds the air in the outdoor field to the indoor field, and the exhaust channel 12 guides the air in the indoor field to the outdoor field, thereby achieving the ventilation. The host driving controller 5 controls the enablement of the air guiding fans 3 and dynamically adjusts operation frequencies and output air volumes of the air guiding fans 3. As shown in FIG. 2, the gas detection module 6 is electrically connected to the host driving controller 5. The gas detection module 6 is configured to detect temperature, humidity and air pollution of the air to generate a detection data. The detection data is transmitted to a networked cloud computing service device 7 through IoT (Internet of Things) communication. The networked cloud computing service device 7 collects and analyzes the detection data, monitors the detection data in real time, and intelligently selects a control instruction to transmit to the gas detection module 6 for controlling the host driving controller 5 to enable the air guiding fans 3 in the intake channel 11 and the exhaust channel 12 and dynamically adjust the operation frequencies and air output volumes of the air guiding fans 3, thereby realizing the gas circulation and heat exchange between the indoor field and the outdoor field. Notably, the air pollution includes at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus and a combination thereof. Notably, in the embodiment, the detection data outputted by the gas detection module 6 can be particulate matters (PM1, PM2.5, PM10), the concentration of carbon dioxide (CO₂), temperature, humidity etc.

As shown in FIG. 2, the gas detection module 6 includes a controlling circuit board 61 and a gas detection main part 62. The gas detection main part 62 detects the air pollution, the concentration of carbon dioxide (CO₂), the temperature and the humidity to generate the detection data. The controlling circuit board 61 collects, calculates and analyzes the detection data to form and output a serial communication (IIC) signal for input, and the networked cloud computing service device 7 receives and analyzes the detection data in real time to output a Universal Asynchronous Transceiver and Transceiver (UART) signal and a General Purpose Input and Output (GP I/O) signal to the host driving controller 5.

The controlling circuit board 61 includes a power conversion component 611, at least one connection interface 612, a microcontroller (MCU) 613 and a wireless communicator 614. The power conversion component 611 provides DC voltage division modulation to output a required DC voltage. The required DC voltage is transmitted through the at least one connection interface 612 to the gas detection main part 62 for enablement and to the host driving controller 5 for enablement. The microcontroller (MCU) 613 is connected through the at least one connection interface 612 to receive the serial communication (IIC) signal for input formed from the detection data and outputted by the gas detection main part 62 so as to calculate and analyze the detection data, and is connected through the at least one connection interface 612 to output the Universal Asynchronous Transceiver and Transceiver (UART) signal and the General Purpose Input and Output (GP I/O) signal for regulation of the host driving controller 5. The wireless communicator 614 receives the detection data and transmits the detection data to the networked cloud computing service device 7 through an external wireless communication. The networked cloud computing service device 7 collects, analyzes and monitors the detection data in real time and intelligently selects a control command, and the control command is received through the wireless communicator 614 and transmitted to the microcontroller (MCU) 613 to output the Universal Asynchronous Transceiver and Transceiver (UART) signal and the General Purpose Input and Output (GP I/O) signal for regulation of the host driving controller 5, so that the host driving controller 5 is regulated to control the enablement of the air guiding fans 3 and dynamically adjust the operation frequencies and the output air volumes of the air guiding fans 3, and to enable a temperature regulation module in the heat exchange core 4 and control the operation efficiency of the temperature regulation module in the heating and cooling exchanging mode. Notably, the output air volume of the air guiding fan 3 is over 500 m³/h, and the noise level of the air guiding fan 3 during operation is 35~50 dB.

The gas detection module 6 further includes a wired communication port 615. The wired communication port 615 is electrically connected to the controlling circuit board 61 through one connection interface 612 for externally connecting to a wired communication for transmission. The detection data is received and externally transmitted to the networked cloud computing service device 7 through the wired communication, the networked cloud computing service device 7 collects, analyzes and monitors the detection data in real time and intelligently selects a control command, and the control command is received through the wired communication port 615 and transmitted to the microcontroller (MCU) 613 to output the Universal Asynchronous Transceiver and Transceiver (UART) signal and the General Purpose Input and Output (GP I/O) signal for regulation of the host driving controller 5, so that the host driving controller 5 is regulated to control the enablement of the air guiding fans 3, and dynamically adjust the operating frequencies and the output air volumes of the air guiding fans 3, and to enable the temperature regulation module in the heat exchange core 4 and control the operation efficiency of the temperature regulation module in the heating and cooling exchanging mode. Notably, the wired communication port 615 is an RS485 port that communicates with the networked cloud computing service device 7 through a wired line connection.

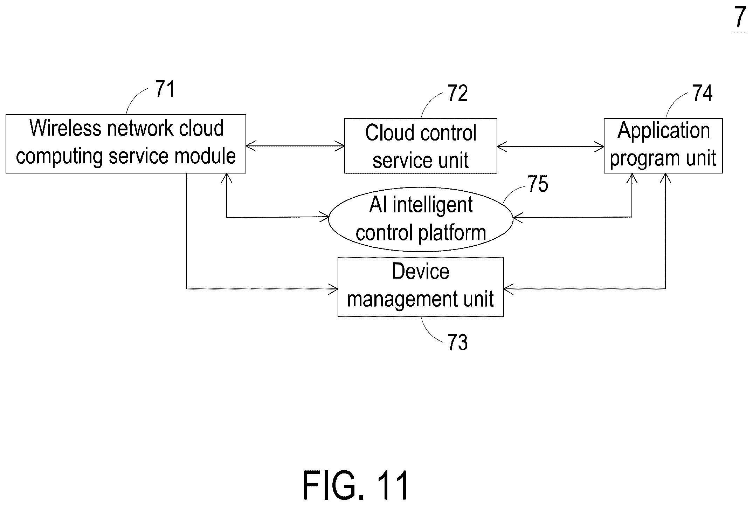

As shown in FIG. 11, the networked cloud computing service device 7 includes a wireless network cloud computing service module 71, a cloud control service unit 72, a device management unit 73, an application program unit 74 and an AI smart calculation platform 75. In the embodiment, the wireless network cloud computing service module 71 receives the detection data related to the air qualities of the outdoor field and the indoor field, receives the communication information of the gas detection module 6 of the intelligent networked energy recovery ventilator, and transmits the control command. The wireless network cloud computing service module 71 receives the detection data related to the air quality of the indoor field and transmits thereof to the cloud control service unit 72 to store and form a big data database of air pollution data. Through performing an artificial intelligence calculation and comparing with the database of air pollution data, the control instruction is sent out and transmitted to the wireless network cloud computing service module 71, and then, the control instruction is further transmitted to the gas detection module 6 of the intelligent networked energy recovery ventilator through the wireless network cloud computing service module 71 to control the enablement of the gas detection module 6.

The device management unit 73 receives the communication information of the gas detection module 6 of the intelligent networked energy recovery ventilator through the wireless network cloud computing service module 71 to manage the user login and device binding, and also provides the management information to the application program unit 74 for system control and management, wherein the management information may include maintenance and management of the intelligent networked energy recovery ventilator, automated abnormal point detection, analysis, treatment and improvement, customer demand feedback, and correction mechanism for software and hardware technology improvement. The application program unit 74 can also display and inform the detection data of air quality information obtained from the cloud control service unit 72, so the user can know the real-time status of the intelligent networked energy recovery ventilator through the mobile phone or the communication device. Moreover, the user can control the operation of the intelligent networked energy recovery ventilator through the application program unit 74 of the mobile phone or the communication device. The AI smart calculation platform 75 receives the detection data of air quality from the gas detection module 6 of the intelligent networked energy recovery ventilator through the IoT communication, analyzes thereof, and generates the control instruction based on the analysis result, so as to achieve the automatic control and optimization of the intelligent networked energy recovery ventilator, thereby automatically adjusting the operation mode of the intelligent networked energy recovery ventilator.

Notably, in the embodiment, the IoT (Internet of Things) communication refers to a collective network, which connects various devices and technologies and helps the devices communicate with the cloud and with each other. Preferably but not exclusively, the IoT communication is a wired communication, which is connected to the networked cloud computing service device 7 via a wired line. Preferably but not exclusively, the IoT communication is a wireless communication for communicating with the networked cloud computing service device 3 via a wireless connection. The wireless communication transmission includes one selected from the group consisting of a Wi-Fi communication, a Bluetooth communication, a radio frequency identification communication, and a near field communication (NFC) communication.

In an embodiment, an implementation of the intelligent networked energy recovery ventilator of the present disclosure is as follows. When the real-time monitored detection data of carbon dioxide (CO₂) is too high, the AI smart calculation platform 75 of the networked cloud computing service device 7 immediately and intelligently selects a control instruction to transmit to the gas detection module 6 for controlling the host driving controller 5 to enable the air guiding fans 3 in the intake channel 11 and the exhaust channel 12. Accordingly, the air in the outdoor field is introduced to pass through the filtering component 2 for filtration and then pass through the heat exchange core 4 for performing heat exchange and entering the indoor field via the other end of the intake channel 11, and further, the circulated air in the indoor field is exhausted through the exhaust channel 12 to pass through the filtering component 2 for filtration and then pass through the heat exchange core 4 for performing heat exchange and exhaling to the outdoor field via the other end of the exhaust channel 12, thereby forming the gas exchange between the outdoor field and the indoor field (as indicated by the arrows in FIG. 1). In the embodiment, through receiving, analysis and calculation of the detection data by the AI smart calculation platform 75 of the networked cloud computing service device 7, it can achieve AI intelligent controlled environmental data analysis for realizing automated operation, real-time monitoring of indoor air quality, and continuous adjustment of indoor temperature and humidity, thereby providing fresh and comfortable air and achieving energy-efficient indoor temperature control.

From the above descriptions, the present disclosure provides an intelligent networked energy recovery ventilator in which through the gas detection module 6 continuously monitors the environment, including temperature, humidity, concentration of carbon dioxide (CO₂), air pollution etc., the AI smart calculation platform 75 of the networked cloud computing service device 7 can receive, analyze and calculate the detection data to achieve AI intelligent controlled environmental data analysis for realizing automated operation and real-time monitoring of indoor air quality, so that when the concentration of carbon dioxide (CO₂) is too high, the fresh air in the outdoor field can be introduced in and filtered for gas exchange. Moreover, indoor temperature and humidity also can be adjusted to provide fresh and comfortable air and achieve energy-efficient indoor temperature control. Accordingly, the environmental status of the indoor field can respond quickly to real-time environmental changes, thereby optimizing system energy efficiency and maintaining optimal air quality.

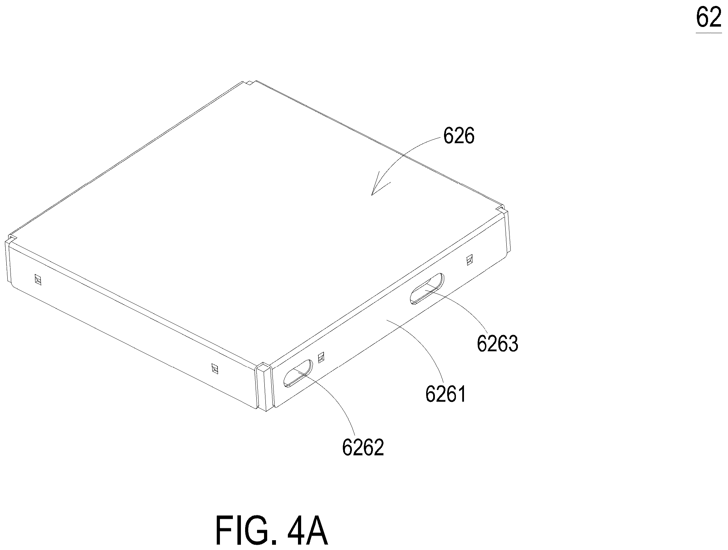

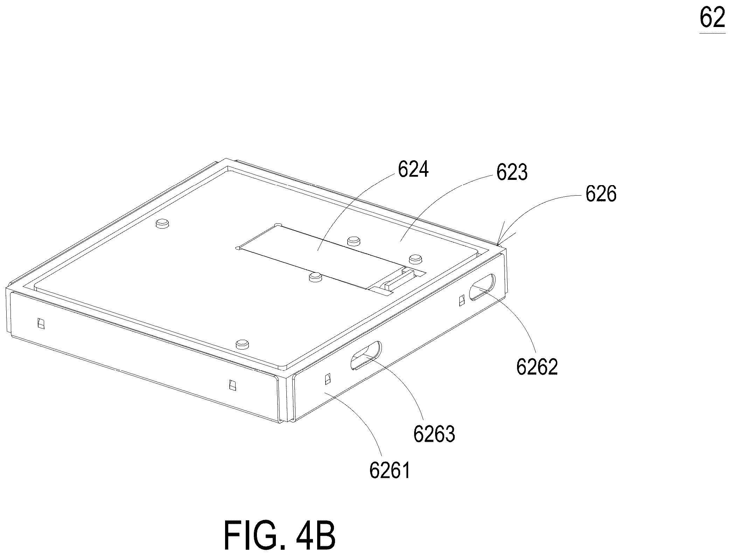

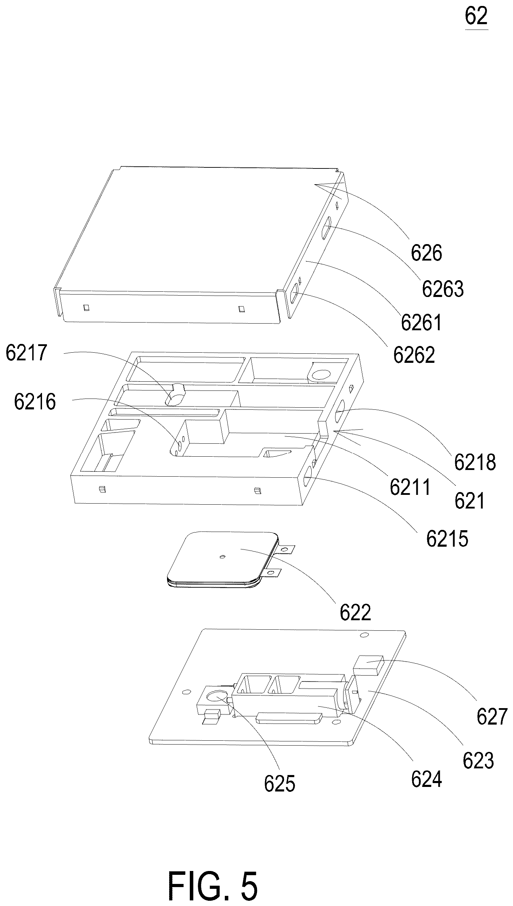

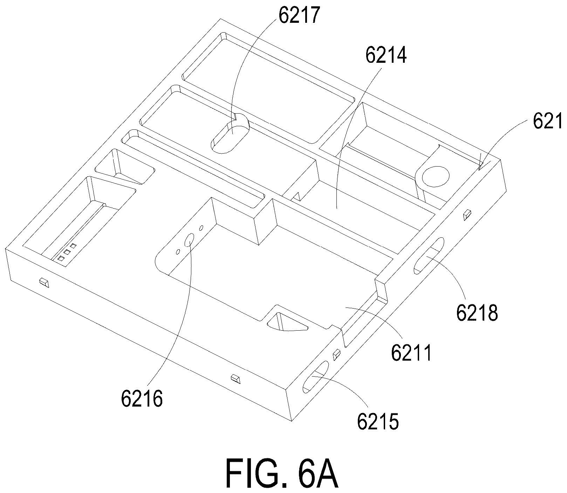

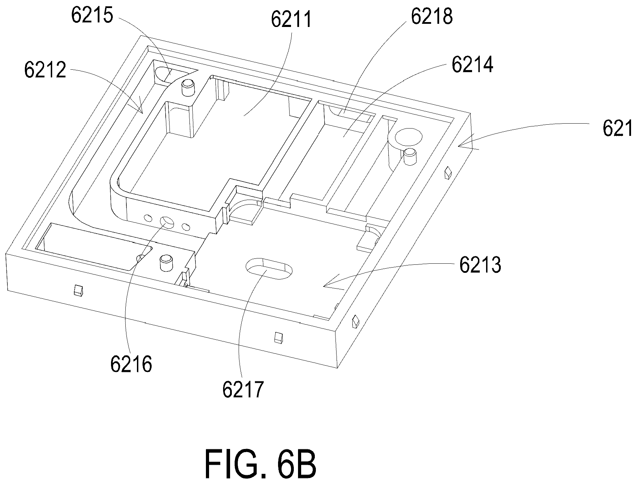

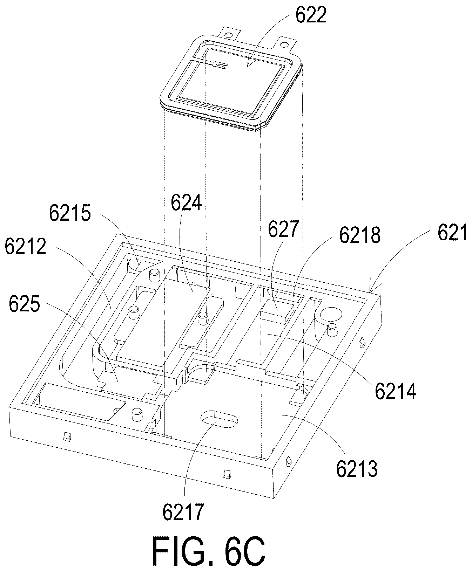

Please refer to FIG. 4A to FIG. 9C. After understanding the overall structure of the intelligent networked energy recovery ventilator of the present disclosure, the detailed structure of the gas detection main part 62 of the gas detection module 6 will be described in detail below.

Please refer to FIG. 4A, FIG. 4B, FIG. 5, FIG. 6A to FIG. 6C and FIG. 7. In the embodiment, the gas detection main part 62 includes a base 621, a piezoelectric actuator 622, a driving circuit board 623, a laser component 624, a particulate sensor 625, an outer cover 626 and a gas sensor 627.

In the embodiment, the base 621 includes a laser loading region 6211, a gas-inlet groove 6212, a gas-guiding-component loading region 6213 and a gas-outlet groove 6214. The gas-inlet groove 6212 includes a gas-inlet 6215 and two lateral walls, the gas-inlet 6215 is in communication with an environment outside the base, and transparent windows 6216 are respectively opened on the two lateral walls and are in communication with the laser loading region 6211. The gas-guiding-component loading region 6213 is in communication with the gas-inlet groove 6212, and a ventilation hole 6217 penetrates a bottom surface of the gas-guiding-component loading region 6213. The gas-outlet groove 6214 is in communication with the ventilation hole 6217, and a gas-outlet 6218 is disposed in the gas-outlet groove 6214. In the embodiment, the outer cover 626 covers the base 621, and includes a side plate 6261. The side plate 6261 has an inlet opening 6262 and an outlet opening 6263. The inlet opening 6262 is spatially corresponding to the gas-inlet 6215 of the base 621, and the outlet opening 6263 is spatially corresponding to the gas-outlet 6218 of the base 621.

In the embodiment, the laser component 624, the particulate sensor 625 and the gas sensor 627 are disposed on and electrically connected to the driving circuit board 623 and located within the base 621. In order to clearly describe and illustrate the positions of the laser component 624 and the particulate sensor 625 in the base 621, the driving circuit board 623 is intentionally omitted. The laser component 624 is accommodated in the laser loading region 6211 of the base 621, and the particulate sensor 625 is accommodated in the gas-inlet groove 6212 of the base 621 and is aligned to the laser component 624. In addition, the laser component 624 is spatially corresponding to the transparent window 6216, therefore, a light beam emitted by the laser component 624 passes through the transparent window 6216 and is irradiated into the gas-inlet groove 6212. A light beam path emitted from the laser component 624 passes through the transparent window 6216 and extends in an orthogonal direction perpendicular to the gas-inlet groove 6212. In the embodiment, a projecting light beam emitted from the laser component 624 passes through the transparent window 6216 and enters the gas-inlet groove 6212 to irradiate the suspended particles contained in the gas passing through the gas-inlet groove 6212. When the suspended particles contained in the gas are irradiated and generate scattered light spots, the scattered light spots are received and calculated by the particulate sensor 625 in the orthogonal direction to obtain the gas detection data. Notably, the laser component 624 emits a parallel light source, and the parallel light source passes through the transparent window 6216.

In the embodiment, the gas sensor 627 is positioned and accommodated in the gas-outlet groove 6214, so as to detect the air pollution introduced into the gas-outlet groove 6214. Preferably but not exclusively, the particulate sensor 625 detects suspended particulates and outputs the detection data. Moreover, the gas sensor 627 includes a volatile-organic-compound sensor, and the volatile-organic-compound sensor detects carbon dioxide (CO₂) or volatile organic compounds (TVOC) in the gas to output the detection data. In an embodiment, the gas sensor 627 is a formaldehyde sensor, and the formaldehyde sensor detects formaldehyde (HCHO) in the gas to output the detection data. In an embodiment, the gas sensor 627 is a bacteria sensor, and the bacteria sensor detects information of bacteria or fungi in the gas to output the detection data. In an embodiment, the gas sensor 627 is a virus sensor, and the virus sensor detects information of virus in the gas to output the detection data. In an embodiment, the gas sensor 627 is a temperature and humidity sensor, and the temperature and humidity sensor detects the temperature and humidity of the gas to output the detection data.

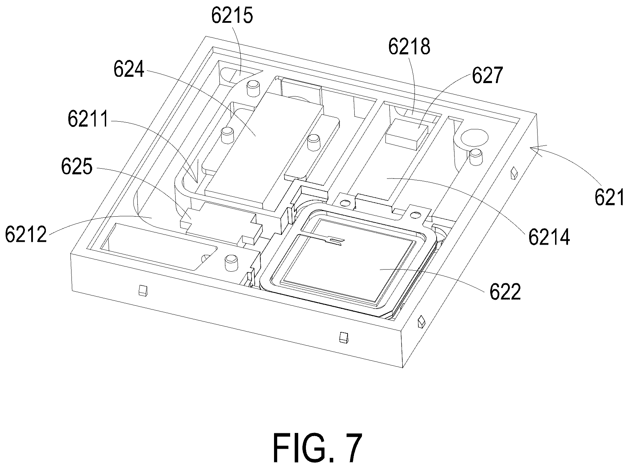

Please refer to FIG. 7. In the embodiment, the piezoelectric actuator 622 is accommodated in the gas-guiding-component loading region 6213 of the base 621. In addition, the gas-guiding-component loading region 6213 of the base 621 is in fluid communication with the gas-inlet groove 6212. When the driving circuit board 623 is covered inside the base 621 and the outer cover 626 is covered outside the base 621, the inlet opening 6262 corresponds to the gas-inlet 6215 of the base 621 to collaboratively define a gas inlet path, and the outlet opening 6263 corresponds to the gas-outlet 6218 of the base 621 to collaboratively define a gas outlet path. When the piezoelectric actuator 622 is enabled, the gas in the gas-inlet groove 6212 is inhaled by the piezoelectric actuator 622, so that the gas flows into the piezoelectric actuator 622, and is transported into the gas-outlet groove 6214 through the ventilation hole 6217 of the gas-guiding-component loading region 6213. Finally, when the gas enters the gas-outlet groove 6214, the piezoelectric actuator 622 continuously transports the gas from the gas inlet path into the gas-outlet groove 6214, and the gas in the gas-outlet groove 6214 is pushed to the gas outlet path and passed through the gas-outlet 6218 and the outlet opening 6263 to discharge to the outside, to achieve the gas transportation at high speed and in large quantities.

After understanding the above structural description of the gas detection main part 62, the detailed structure of the piezoelectric actuator 622 will be described in detail below.

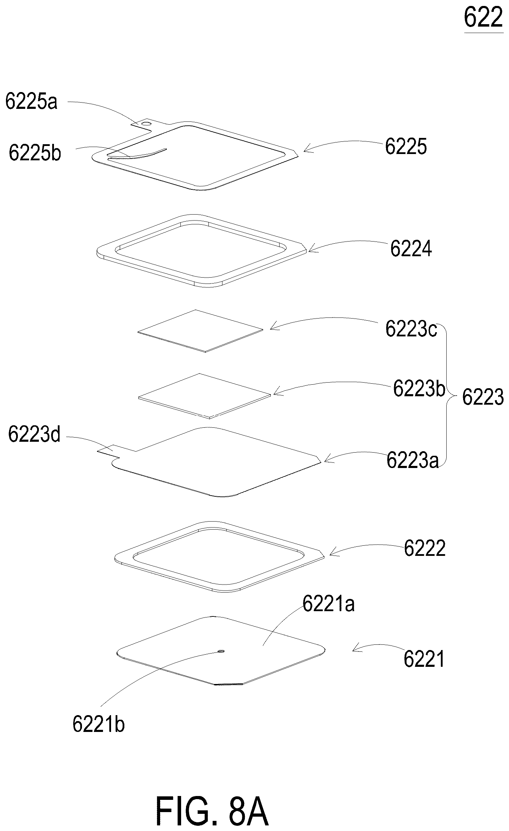

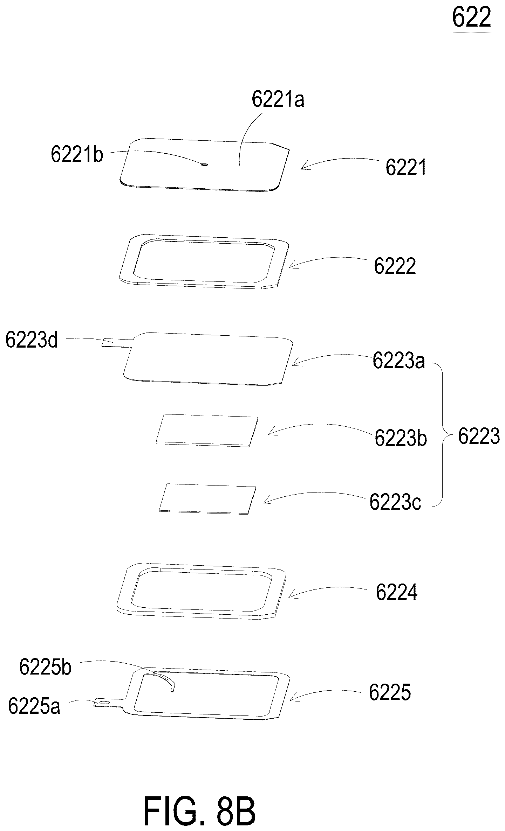

Please refer to FIG. 8A and FIG. 8B. In the embodiment, the piezoelectric actuator 622 includes a gas-injection plate 6221, a chamber frame 6222, an actuator element 6223, an insulation frame 6224 and a conductive frame 6225. In the embodiment, the gas-injection plate 6221 is made by a flexible material and includes a suspension plate 6221a and a hollow aperture 6221b. The suspension plate 6221a is a sheet structure and is permitted to undergo a bending deformation. Preferably but not exclusively, the shape and the size of the suspension plate 6221a correspond to the inner edge of the gas-guiding-component loading region 6215, but not limited thereto. The hollow aperture 6221b passes through a center of the suspension plate 6221a, so as to allow the gas to flow therethrough. Preferably but not exclusively, in the embodiment, the shape of the suspension plate 6221a is selected from the group consisting of a square, a circle, an ellipse, a triangle and a polygon, but not limited thereto.

In the embodiment, the chamber frame 6222 is carried and stacked on the gas-injection plate 6221. In addition, the shape of the chamber frame 6222 is corresponding to the gas-injection plate 6221. The actuator element 6223 is carried and stacked on the chamber frame 6222. A resonance chamber 6226 is collaboratively defined by the actuator element 6223, the chamber frame 6222 and the suspension plate 6221a and is formed between the actuator element 6223, the chamber frame 6222 and the suspension plate 6221a. The insulation frame 6224 is carried and stacked on the actuator element 6223 and the appearance of the insulation frame 6224 is similar to that of the chamber frame 6222. The conductive frame 6225 is carried and stacked on the insulation frame 6224, and the appearance of the conductive frame 6225 is similar to that of the insulation frame 6224. In addition, the conductive frame 6225 includes a conducting pin 6225a and a conducting electrode 6225b. The conducting pin 6225a is extended outwardly from an outer edge of the conductive frame 6225, and the conducting electrode 6225b is extended inwardly from an inner edge of the conductive frame 6225.

Moreover, the actuator element 6223 further includes a piezoelectric carrying plate 6223a, an adjusting resonance plate 6223b and a piezoelectric plate 6223c. The piezoelectric carrying plate 6223a is carried and stacked on the chamber frame 6222. The adjusting resonance plate 6223b is carried and stacked on the piezoelectric carrying plate 6223a. The piezoelectric plate 6223c is carried and stacked on the adjusting resonance plate 6223b. The adjusting resonance plate 6223b and the piezoelectric plate 6223c are accommodated in the insulation frame 6224. The conducting electrode 6225b of the conductive frame 6225 is electrically connected to the piezoelectric plate 6223c. In the embodiment, the piezoelectric carrying plate 6223a and the adjusting resonance plate 6223b are made by conductive materials. The piezoelectric carrying plate 6223a includes a piezoelectric pin 6223d. The piezoelectric pin 6223d and the conducting pin 6225a are electrically connected to a driving circuit (not shown) of the driving circuit board 623, so as to receive a driving signal, such as a driving frequency and a driving voltage. Through this structure, a circuit is formed by the piezoelectric pin 6223d, the piezoelectric carrying plate 6223a, the adjusting resonance plate 6223b, the piezoelectric plate 6223c, the conducting electrode 6225b, the conductive frame 6225 and the conducting pin 6225a for transmitting the driving signal. Moreover, the insulation frame 6224 is insulated between the conductive frame 6225 and the actuator element 6223, so as to avoid the occurrence of a short circuit. Thereby, the driving signal is transmitted to the piezoelectric plate 6223c. After receiving the driving signal, the piezoelectric plate 6223c deforms due to the piezoelectric effect, and the piezoelectric carrying plate 6223a and the adjusting resonance plate 6223b are further driven to generate the bending deformation in the reciprocating manner.

Furthermore, in the embodiment, the adjusting resonance plate 6223b is located between the piezoelectric plate 6223c and the piezoelectric carrying plate 6223a and served as a cushion between the piezoelectric plate 6223c and the piezoelectric carrying plate 6223a. Thereby, the vibration frequency of the piezoelectric carrying plate 6223a is adjustable. Basically, the thickness of the adjusting resonance plate 6223b is greater than the thickness of the piezoelectric carrying plate 6223a, and the vibration frequency of the actuator element 6223 can be adjusted by adjusting the thickness of the adjusting resonance plate 6223b. In the embodiment, the gas-injection plate 6221, the chamber frame 6222, the actuator element 6223, the insulation frame 6224 and the conductive frame 6225 are stacked and positioned in the gas-guiding-component loading region 6213 sequentially, so that the piezoelectric actuator 622 is supported and positioned in the gas-guiding-component loading region 6213. A clearance 6221c is defined between the suspension plate 6221a of the gas-injection plate 6221 and an inner edge of the gas-guiding-component loading region 6213 for gas flowing therethrough.

In the embodiment, a flowing chamber 6227 is formed between the gas-injection plate 6221 and the bottom surface of the gas-guiding-component loading region 6213. The flowing chamber 6227 is in communication with the resonance chamber 6226 between the actuator element 6223, the chamber frame 6222 and the suspension plate 6221a. By controlling the vibration frequency of the gas in the resonance chamber 6226 to be close to the vibration frequency of the suspension plate 6221a, the Helmholtz resonance effect is generated between the resonance chamber 6226 and the suspension plate 6221a, so as to improve the efficiency of gas transportation. When the piezoelectric plate 6223c is moved away from the bottom surface of the gas-guiding-component loading region 6213, the suspension plate 6221a of the gas-injection plate 6221 is driven to move away from the bottom surface of the gas-guiding-component loading region 6213 by the piezoelectric plate 6223c. In that, the volume of the flowing chamber 6227 is expanded rapidly, the internal pressure of the flowing chamber 6227 is decreased to form a negative pressure, and the gas outside the piezoelectric actuator 622 is inhaled through the clearance 6221c and enters the resonance chamber 6226 through the hollow aperture 6221b. Consequently, the pressure in the resonance chamber 6226 is increased to generate a pressure gradient. When the suspension plate 6221a of the gas-injection plate 6221 is driven by the piezoelectric plate 6223c to move toward the bottom surface of the gas-guiding-component loading region 6213, the gas in the resonance chamber 6226 is discharged out rapidly through the hollow aperture 6221b, and the gas in the flowing chamber 6227 is compressed, thereby the converged gas is quickly and massively ejected out of the flowing chamber 6227 under the condition close to an ideal gas state of the Benulli's law, and transported to the ventilation hole 6217 of the gas-guiding-component loading region 6213.

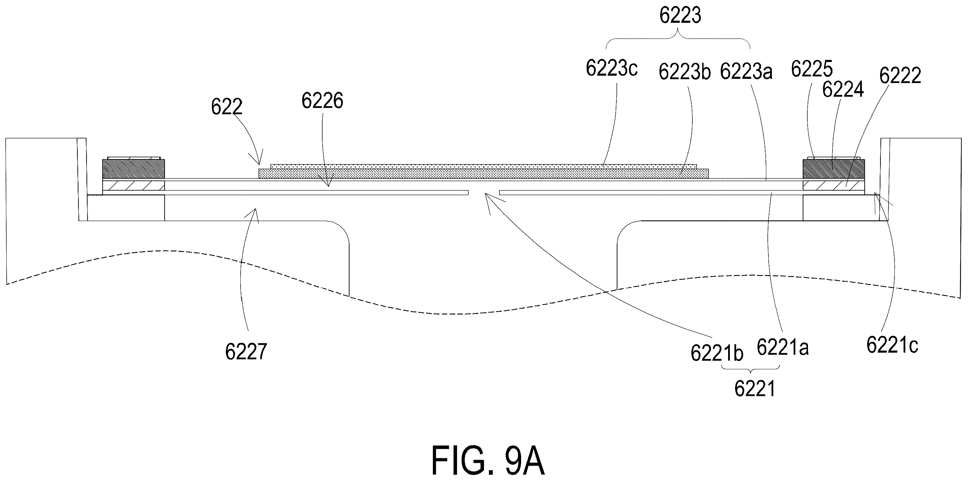

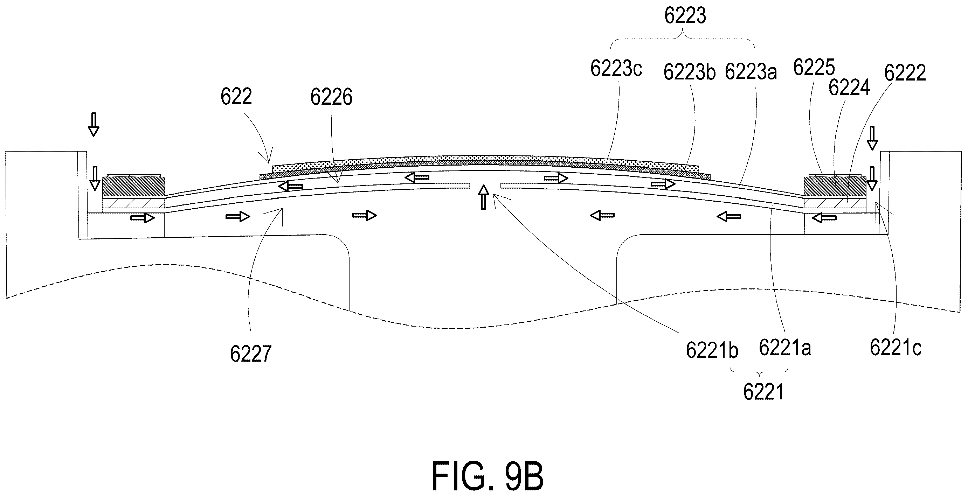

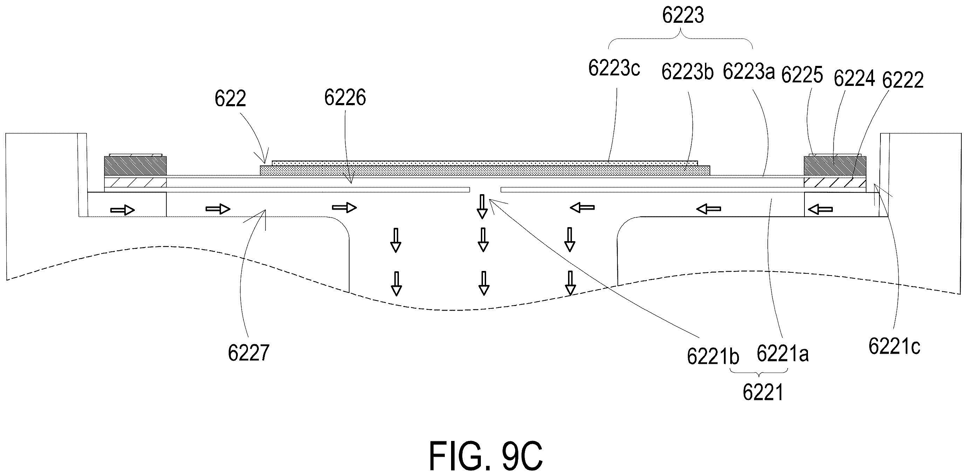

By repeating the above operation steps shown in FIG. 9B and FIG. 9C, the piezoelectric plate 6223c is driven to generate the bending deformation in a reciprocating manner. According to the principle of inertia, since the gas pressure inside the resonance chamber 6226 is lower than the equilibrium gas pressure after the converged gas is ejected out, the gas is introduced into the resonance chamber 6226 again. Moreover, the vibration frequency of the gas in the resonance chamber 6226 is controlled to be close to the vibration frequency of the piezoelectric plate 6223c, so as to generate the Helmholtz resonance effect to achieve the gas transportation at high speed and in large quantities.

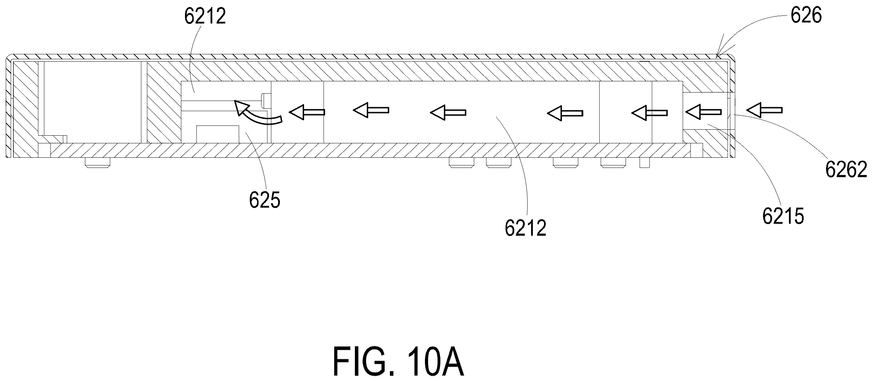

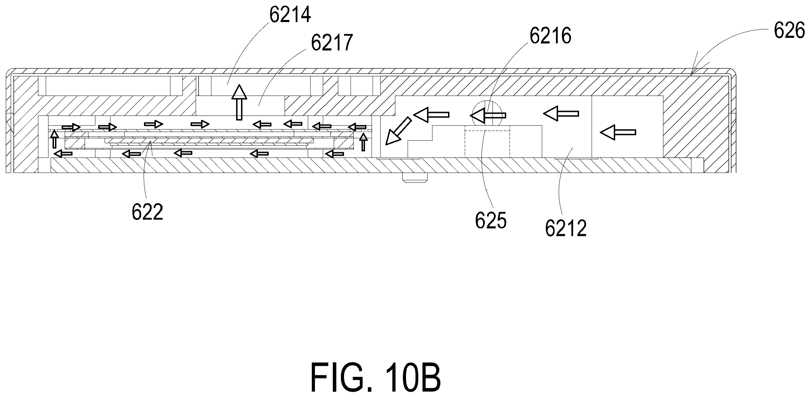

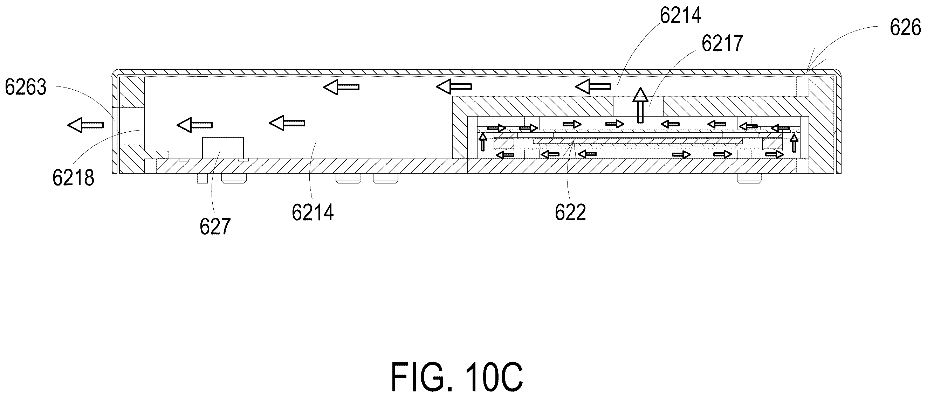

Please refer to FIG. 10A to FIG. 10C. The gas is inhaled through the inlet opening 6262 on the outer cover 626, flows into the gas-inlet groove 6212 of the base 621 through the gas-inlet 6215, and is transported to the position of the particulate sensor 625. In addition, the piezoelectric actuator 622 is enabled to continuously inhale the gas into the gas inlet path so as to facilitate the gas outside to be introduced rapidly, flow stably, and transported above the particulate sensor 625. At this time, a projecting light beam emitted from the laser component 624 passes through the transparent window 6216 to irritate the suspended particles contained in the gas flowing above the particulate sensor 625 in the gas-inlet groove 6212. When the suspended particles contained in the gas are irradiated and generate scattered light spots, the scattered light spots are received and calculated by the particulate sensor 625 for obtaining related information about the sizes and the concentration of the suspended particles contained in the gas. Moreover, the gas above the particulate sensor 625 is continuously driven and transported by the piezoelectric actuator 622, flows into the ventilation hole 6217 of the gas-guiding-component loading region 6213, and is transported to the gas-outlet groove 6214. At last, after the gas flows into the gas outlet groove 6214, the gas is continuously transported into the gas-outlet groove 6214 by the piezoelectric actuator 622, and thus the gas in the gas-outlet groove 6214 is pushed to discharge through the gas-outlet 6218 and the outlet opening 6263, to achieve the gas transportation at high speed and in large quantities.

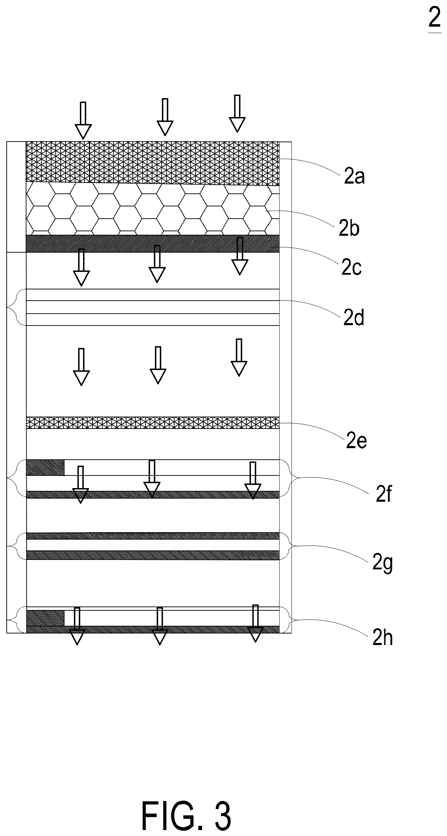

Please again refer to FIG. 3. The filtering component 2 of the present disclosure can be a combination of various implementation forms. Preferably but not exclusively, in an embodiment, the filtering component 2 is a filter screen 2a of minimum filtration efficiency value (MREV) 8 or above, or a filter screen 2a of high-efficiency particulate air (HEPA) grade or above, which is configured to absorb the chemical smoke, the bacteria, the dust particles and the pollen contained in the air pollution, so that the air pollution introduced is filtered and purified to achieve the effect of filtering and purification. Notably, in the present disclosure, the filter screen is of high efficiency particulate air (HEPA) 10 or above, and has a dust holding capacity greater than 12,000 mg. Alternatively, the filter screen 2a is of ULPA14 grade, so as to improve filtration efficiency and meet higher cleanliness requirements. In some specific embodiments, the filtering component 2 is further combined with physical or chemical materials to provide a sterilization effect for air pollution passing therethrough, and the airflow path directions of the air guiding fans 3 are the directions indicated by the arrows. In an embodiment, the filtering component 2 includes a decomposition layer coated thereon to sterilize air pollution passing therethrough in chemical means. Preferably but not exclusively, the decomposition layer includes an activated carbon 2b configured to remove organic and inorganic substances in air pollution, and remove colored and odorous substances. Notably, the activated carbon 2b has a formaldehyde absorption capacity greater than 1,500 mg. Moreover, in some embodiments, the filtering component 2 is combined with a light irradiation element to sterilize air pollution passing therethrough in chemical means. Preferably but not exclusively, the light irradiation element is a photo-catalyst unit including a photo catalyst 2c and an ultraviolet lamp 2d for improving the removal efficiency of pollutants and allergens in the air. When the photo catalyst 2c is irradiated by the ultraviolet lamp 2d, the light energy is converted into the chemical energy, thereby decomposes harmful gases and disinfects bacteria contained in the air pollution, so as to achieve the effects of filtering and sterilization. Notably, the power of the ultraviolet lamp 2d in the present disclosure is more than 120 mw. In an embodiment, the light irradiation element is a photo-plasma unit including a nanometer irradiation tube 2e. When the introduced air pollution is irradiated by the nanometer irradiation tube 2e, the oxygen molecules and water molecules contained in the air pollution are decomposed into high oxidizing photo-plasma, and an ion flow capable of destroying organic molecules is generated. In that, volatile formaldehyde, volatile toluene and volatile organic compounds (VOC) contained in the air pollution are decomposed into water and carbon dioxide for improving the removal efficiency of pollutants and allergens in the air and thus achieving the effects of filtration and sterilization. Furthermore, in some embodiments, the filtering component 2 is combined with a decomposition unit to sterilize air pollution passing therethrough in chemical means. Preferably but not exclusively, the decomposition unit is a negative ion unit 2f. It makes the suspended particles carrying with positive charges in the air pollution to adhere thereto, so as to improve the removal efficiency of pollutants and allergens in the air and thus achieve the effects of filtration and sterilization. Preferably but not exclusively, the decomposition unit is a plasma ion unit 2g. The oxygen molecules and water molecules contained in the air pollution are decomposed into positive hydrogen ions (H⁺) and negative oxygen ions (O²⁻) by the plasma ion. The substances attached with water around the ions are adhered on the surface of viruses and bacteria and converted into OH radicals with extremely strong oxidizing power, thereby removing hydrogen (H) from the protein on the surface of viruses and bacteria, and thus decomposing (oxidizing) the protein, so as to decompose and eliminate pollutants, allergens and microorganisms in the air pollution and improve the cleanliness of the air, thereby achieving the effects of filtration and sterilization for the introduced air pollution. Preferably but not exclusively, the decomposition unit is an electrostatic filtering unit 2h. The electrostatic force thereof is used to capture and remove suspended particles (such as dust, pollen, bacteria and other pollutants) in the air.

From the above descriptions, the present disclosure provides an intelligent networked energy recovery ventilator with the following advantages in the specific implementation. Intelligent heat recycle and regulation: The gas exchange can be regulated through adjusting operations of the air guiding fans in the intake channel and the exhaust channel based on the detection data of temperature and humidity, wherein the air guiding fan in the intake channel suctions the air in the outdoor field, so that the air is introduced into the intake channel, passes through the heat exchange core for heat exchange, and then is guided into the indoor field, and the air guiding fan in the exhaust channel suctions the air in the indoor field, so that the air is introduced into the exhaust channel, passes through the heat exchange core for heat exchange, and then is exhaled to the outdoor field, thereby improving gas exchanging efficiency and recycling heat and maintaining a stable indoor temperature. Automated control of gas exchange and air volume: When the concentration of CO₂ or PM2.5 exceeds the threshold, the ventilation volume is automatically increased and the speed of fan is also adjusted for ensuring the indoor air fresh. Intelligent filtering mode: If the outdoor air quality is poor, it is automatically adjusted to a low ventilation mode, and the filtering component is used to purify the air entering the indoor field. Remote control and monitoring: Users can view indoor and outdoor environmental conditions and remotely operate the equipment through the networked cloud computing service device or mobile application. Energy-saving mode: When indoor and outdoor temperature and humidity are similar or air qualities meets standards, operation frequency is automatically reduced to enter the energy-saving mode, so as to reduce power consumption.

In summary, the present disclosure provides an intelligent networked energy recovery ventilator, which includes a built-in gas detection module for detecting air qualities indoor and outdoor in real time. Moreover, the gas detection module is equipped with cloud connection capability, so that the user can remotely monitor environmental condition and control the equipment. Furthermore, through IoT communication, the ventilation volume and heat recovery efficiency can be dynamically adjusted based on detection data, such as temperature, humidity and air quality, thereby maintaining an optimal indoor air quality.

## Claims

1. An intelligent networked energy recovery ventilator, **characterized by** comprising:
a main body (1) comprising an intake channel (11) and an exhaust channel (12);
at least one filtering component (2) disposed at an entrance of the intake channel (11) and/or an entrance of the exhaust channel (12);
at least one air guiding fan (3) disposed at a rear side of a filtering component (2) of the at least one filtering component (2) at the entrance of the intake channel (11) and/or at a rear side of a filtering component (2) of the at least one filtering component (2) at the entrance of the exhaust channel (12), wherein the at least one air guiding fan (3) is configured to guide a gas to pass through the at least one filtering component (2) for purification and to achieve a gas exchange between an indoor field and an outdoor field;
a heat exchange core (4) disposed in the main body (1) and respectively communicated with the intake channel (11) and the exhaust channel (12), wherein the intake channel (11) and the exhaust channel (12) are not communicated with each other, the intake channel (11) is separated by the heat exchange core (4) to communicate with the indoor field through one end thereof and with the outdoor field through the other end thereof, and the exhaust channel (12) is separated by the heat exchange core (4) to communicate with the indoor field through one end thereof and with the outdoor field through the other end thereof, so that a gas is guided to pass through the heat exchange core (4) for achieving a heat exchange, and wherein the intake channel (11) guides a gas from the outdoor field to the indoor field, and the exhaust channel (12) guides a gas from the indoor field to the outdoor field, so as to achieve the gas exchange between the indoor field and the outdoor field;
at least one host driving controller (5) controlling an enablement of the at least one air guiding fan (3), and dynamically adjusting an operation frequency and an output air volume of the at least one air guiding fan (3); and
at least one gas detection module (6) electrically connected to the at least one host driving controller (5), wherein the at least one gas detection module (6) detects a temperature, a humidity and an air pollution in an air to generate a detection data, the detection data is transmitted to a networked cloud computing service device (7) through IoT communication, and the networked cloud computing service device (7) collects and analyzes the detection data in real time, intelligently selects a control instruction according to the detection data, and transmits the control instruction to the at least one gas detection module (6), for controlling the at least one host driving controller (5) to enable the at least one air guiding fan (3) in the intake channel (11) and/or the exhaust channel (12) and dynamically adjust the operation frequency and the output air volume of the at least one air guiding fan (3), thereby achieving the gas exchange and the heat exchange between the indoor field and the outdoor field.

2. The intelligent networked energy recovery ventilator according to claim 1, wherein the networked cloud computing service device (7) comprises an AI smart calculation platform (75), and the AI smart calculation platform (75) collects, analyzes and monitors the detection data in real time, intelligently selects the control instruction, and transmits the control instruction to the at least one gas detection module (6) for controlling the at least one host driving controller (5) to enable the at least one air guiding fan (3) and dynamically adjust the operation frequency and the output air volume of the at least one air guiding fan (3), and for enabling a temperature regulation module in the heat exchange core (4) and controlling an operation efficiency of the temperature regulation module in a heating and cooling exchanging mode.

3. The intelligent networked energy recovery ventilator according to claim 1, wherein each of the at least one gas detection module (6) comprises a gas detection main part (62) and a controlling circuit board (61), the gas detection main part (62) detects the humidity, the temperature and the air pollution in the air to generate the detection data, the controlling circuit board (61) collects, calculates, and analyzes the detection data to form and output a serial communication (IIC) signal for input, and the networked cloud computing service device (7) receives and analyzes the detection data in real time to output a Universal Asynchronous Transceiver and Transceiver (UART) signal and a General Purpose Input and Output (GP I/O) signal to the at least one host driving controller (5).

4. The intelligent networked energy recovery ventilator according to claim 3, wherein the controlling circuit board (61) comprises:
at least one connection interface (612);
a power conversion component (611), providing DC voltage division modulation to output a required DC voltage, wherein the required DC voltage is transmitted through the at least one connection interface (612) to the gas detection main part (62) for enablement and to the host driving controller (5) for enablement;
a microcontroller (MCU) (613), connected to the gas detection main part (62) through the at least one connection interface (612) to receive the serial communication (IIC) signal for input formed from the detection data so as to calculate and analyze the detection data, and connected through the at least one connection interface (612) to output the Universal Asynchronous Transceiver and Transceiver (UART) signal and the General Purpose Input and Output (GP I/O) signal for regulation; and
a wireless communicator (614), receiving the detection data and transmitting the detection data to the networked cloud computing service device (7) through an external wireless communication, wherein the networked cloud computing service device (7) collects, analyzes and monitors the detection data in real time and intelligently selects the control instruction, and the control instruction is received through the wireless communicator (614) and transmitted to the microcontroller (MCU) (613) to output the Universal Asynchronous Transceiver and Transceiver (UART) signal and the General Purpose Input and Output (GP I/O) signal for regulation of the at least one host driving controller (5), so that the at least one host driving controller (5) is regulated to enable the at least one air guiding fan (3) and dynamically adjust the operation frequency and the output air volume of the at least one air guiding fan (3), and to enable a temperature regulation module in the heat exchange core (4) and control an operation efficiency of the temperature regulation module in a heating and cooling exchanging mode.

5. The intelligent networked energy recovery ventilator according to claim 4, the controlling circuit board (61) further comprises a wired communication port (615), wherein the wired communication port (615) is electrically connected to the controlling circuit board (61) through the at least one connection interface (612) for externally connecting to a wired communication for transmission, wherein the detection data is received and externally transmitted to the networked cloud computing service device (7) through the wired communication, the networked cloud computing service device (7) collects, analyzes and monitors the detection data in real time and intelligently selects the control instruction, and the control instruction is received through the wired communication port (615) and transmitted to the microcontroller (MCU) (613) to output the Universal Asynchronous Transceiver and Transceiver (UART) signal and the General Purpose Input and Output (GP I/O) signal for regulation of the at least one host driving controller (5), so that the at least one host driving controller (5) is regulated to enable the at least one air guiding fan (3) and dynamically adjust the operation frequency and the output air volume of the at least one air guiding fan (3), and to enable the temperature regulation module and control the operation efficiency of the temperature regulation module in the heating and cooling exchanging mode, wherein the wired communication port (615) is an RS485 port that communicates with the networked cloud computing service device (7) through a wired line connection.

6. The intelligent networked energy recovery ventilator according to claim 1, wherein the output air volume of each of the at least one air guiding fan (3) is over 500 m³/h, and a noise level of the at least one air guiding fan (3) during operation is 35~50 dB.

7. The intelligent networked energy recovery ventilator according to claim 1, wherein the IoT communication is a wireless communication for communicating with the networked cloud computing service device (7) via a wireless connection, or a wired communication for communicating with the networked cloud computing service device (7) via a wired connection, and the wireless communication is one selected from the group consisting of a Wi-Fi communication, a Bluetooth communication, a radio frequency identification communication and a near field communication (NFC).

8. The intelligent networked energy recovery ventilator according to claim 2, wherein when the detection data of carbon dioxide (CO₂) is too high, the AI smart calculation platform (75) of the networked cloud computing service device (7) immediately and intelligently selects the control instruction to transmit to the gas detection module (6) for controlling the at least one host driving controller (5) to enable the at least one air guiding fan (3) in the intake channel (11) and/or in the exhaust channel (12), so that the air in the outdoor field is introduced to pass through the at least one filtering component (2) for filtration and then pass through the heat exchange core (4) for performing the heat exchange and entering the indoor field via the other end of the intake channel (11), and further, a circulated air in the indoor field is exhausted through the exhaust channel (12) to pass through the at least one filtering component (2) for filtration and then pass through the heat exchange core (4) for performing the heat exchange and exhaling to the outdoor field via the other end of the exhaust channel (12), thereby forming the gas exchange between the outdoor field and the indoor field.

9. The intelligent networked energy recovery ventilator according to claim 1, wherein the at least one filtering component (2) is a filter screen (2a) of minimum filtration efficiency value (MREV) 8 or above.

10. The intelligent networked energy recovery ventilator according to claim 1, wherein the at least one filtering component (2) is a filter screen (2a) of high-efficiency particulate air (HEPA) grade or above, or a filter screen (2a) of high efficiency particulate air (HEPA) 10 or above, and has a dust holding capacity greater than 12,000 mg.

11. The intelligent networked energy recovery ventilator according to claim 1, wherein the at least one filtering component (2) is a filter screen (2a) of ULPA14 grade.

12. The intelligent networked energy recovery ventilator according to claim 1, wherein the at least one filtering component (2) is combined with a decomposition layer coated thereon to sterilize in chemical means for the air pollution passing therethrough, the decomposition layer is an activated carbon (2b), and the activated carbon (2b) has a formaldehyde absorption capacity greater than 1500 mg.

13. The intelligent networked energy recovery ventilator according to claim 1, wherein the at least one filtering component (2) is combined with a light irradiation element to sterilize in chemical means for the air pollution passing therethrough, and the light irradiation element is a photo-catalyst unit including a photo catalyst (2c) and an ultraviolet lamp (2d), or a photo-plasma unit including a nanometer irradiation tube (2e), wherein the ultraviolet lamp (2d) has a power greater than 120mW.

14. The intelligent networked energy recovery ventilator according to claim 1, wherein the at least one filtering component (2) is combined with a decomposition unit to sterilize in chemical means for the air pollution passing therethrough.

15. The intelligent networked energy recovery ventilator according to claim 14, wherein the decomposition unit is a negative ion unit (2f), a plasma ion unit (2g) or an electrostatic filtering unit (2h).
